**Europäisches Patentamt**

**European Patent Office**

**Office européen des brevets**

(11) Veröffentlichungsnummer : **0 374 699 B1**

(12) **EUROPÄISCHE PATENTSCHRIFT**

(45) Veröffentlichungstag der Patentschrift :
**25.11.92 Patentblatt 92/48**

(21) Anmeldenummer : **89122942.9**

(22) Anmeldetag : **12.12.89**

(51) Int. Cl.$^5$ : **C07C 43/13**, C07C 41/03,
C07C 41/26, C07C 41/42

(54) **Verfahren zur Herstellung von Polyglycerinen.**

(30) Priorität : **19.12.88 DE 3842692**

(43) Veröffentlichungstag der Anmeldung :
**27.06.90 Patentblatt 90/26**

(45) Bekanntmachung des Hinweises auf die
Patenterteilung :
**25.11.92 Patentblatt 92/48**

(84) Benannte Vertragsstaaten :
**AT BE CH DE ES FR GB GR IT LI LU NL SE**

(56) Entgegenhaltungen :
**DE-A- 3 410 520**
**DE-C- 510 422**
**US-A- 2 520 670**
**US-A- 4 053 525**

(73) Patentinhaber : **DEUTSCHE SOLVAY-WERKE
GMBH
Langhansstrasse 6 Postfach 11 02 70
W-5650 Solingen 11 (DE)**

(72) Erfinder : **Jakobson, Gerald, Dr. Dipl.-Chem.
Willinger Weg 21
W-4134 Rheinberg 2 (DE)**
Erfinder : **Siemanowski, Werner, Dr.
Dipl.-Chem.
Am Annaberg 18
W-4134 Rheinberg 1 (DE)**

(74) Vertreter : **Seiler, Siegfried
Langhansstrasse 6 Postfach 11 02 70
W-5650 Solingen 11 (DE)**

EP 0 374 699 B1

**Beschreibung**

Die vorliegende Erfindung betrifft ein Verfahren zur Herstellung von Polyglycerinen (mit mehr als 50 Gew.-% Diglycerin), die arm an cyclischen Komponenten sind, durch Umsetzung von Glycerin mit Chlorhydrinen.

Ein derartiges Verfahren ist bereits aus der US-PS 25 20 670 bekannt, bei dem Glycerin mit Glycerin-α-Monochlorhydrin in Gegenwart von konzentriertem Alkali bei erhöhter Temperatur zu einem Gemisch von Polyglycerinen umgesetzt wird. Dieses Verfahren hat den Nachteil der relativ langen Reaktionsdauer, des hohen Anteiles an Polyglycerinen und daß nach Beendigung der Reaktion das Reaktionsgemisch mit niederen aliphatischen Alkoholen aufgearbeitet werden muß.

Angaben über die erzielten Ausbeuten an Polyglycerinen sowie über deren Gehalt an cyclischen Komponenten werden nicht gemacht.

Aufgabe der vorliegenden Erfindung war es daher, ein Verfahren zu finden, mit dessen Hilfe Polyglycerine (mit mehr als 50 Gew.-% Diglycerin) in guten Ausbeuten und mit nur geringem Anteil an cyclischen Komponenten erhalten werden. Hierbei sollte gleichzeitig eine Isolierung der Zwischenprodukte (Chlorhydrinethermischung) nicht erforderlich sein sowie aus Umweltschutzgründen eine Aufarbeitung der Endprodukte durch Behandlung mit organischen Lösungsmitteln vermieden werden. Darüber hinaus sollte das Verfahren auch bei niedrigeren Temperaturen durchgeführt werden können.

Erfindungsgemäß wurde festgestellt, daß diese Aufgabe dadurch gelöst wird, daß Glycerin mit Epichlorhydrin (anstelle von Chlorhydrin) bei Temperaturen von 20 bis 140 °C, vorzugsweise von 60 °C bis 100 °C, in Gegenwart eines sauren Katalysators und in einem Molverhältnis von Glycerin zu Epichlorhydrin von 10 : 1 bis 1 : 1, vorzugsweise 6 : 1 bis 1,4 : 1, umgesetzt und das erhaltene, nicht aufgetrennte Reaktionsgemisch und/oder das weitgehend von überschüssigem Glycerin befreite Reaktionsgemisch bei einer Temperatur von 50 °C bis 120°C, vorzugsweise von 60 °C bis 95 °C, entsprechend dem Gehalt des Reaktionsgemisches an organisch gebundenem Chlor mit einem alkalisch reagierenden Medium, vorzugsweise einer alkalisch reagierenden wäßrigen Lösung umgesetzt wird, das Reaktionsgemisch nach Wasserzugabe über einen oder mehrere Kationenaustauscher und nachfolgend unter Verwendung von Anionenaustauschern entsalzt, durch Destillation entwässert und das Glycerin-Polyglyceringemisch durch fraktionierte Destillation in Glycerin, Diglycerin und höhere Polyglycerine aufgetrennt wird. Vorteilhafte Molverhältnisse bei der Umsetzung von Glycerin mit Epichlorhydrin sind auch Molverhältnisse von 3 : 1 bis 1 : 1, oder 2,0 : 1 bis 1,5 : 1. Besonders zweckmäßig sind auch Umsetzungstemperaturen von 80 bis 95 °C.

Vorteilhaft wird bei der Umsetzung von Glycerin als saurer Katalysator eine Säure, vorzugsweise Schwefelsäure, Perchlorsäure, Phosphorsäure und/oder phosphorige Säure und/oder Lewis-Säure, verwendet.

Die als saurer Katalysator verwendete Säure wird in einer Konzentration von 0,1 bis 2,0 Gew.-%, vorzugsweise von 0,5 bis 1,2 Gew.-%, bezogen auf das eingesetzte Epichlorhydrin, zugegeben.

Nach einer vorteilhaften Ausführungsform der Erfindung wird das Reaktionsgemisch durch Wasserzugabe auf eine etwa 70 -40 gew.-%ige, vorzugsweise 60 - 50 gew.-%ige, Lösung verdünnt und bei Temperaturen von 30°C bis 90 °C, vorzugsweise von 40 °C bis 70 °C, über eine Kombination aus stark sauren Kationenaustauschern und nachfolgenden schwach basischen Anionenaustauschern entsalzt.

Nach einer bevorzugten Ausführungsform wird zu dem nicht aufgetrennten Reaktionsgemisch aus der Umsetzung von Epichlorhydrin mit Glycerin eine alkalisch reagierende Alkalicarbonatlösung, vorzugsweise eine konzentrierte Sodalösung, gegeben.

Vorteilhaft wird das nicht aufgetrennte Reaktionsgemisch aus der Umsetzung von Epichlorhydrin und Glycerin zu einer alkalisch reagierenden Alkalicarbonatlösung gegeben, wobei das äquivalente Verhältnis von Alkalicarbonat zu dem Gehalt an organisch gebundenem Chlor 1 : 1 bis 1,2 : 1, vorzugsweise 1,05 : 1 bis 1,1 : 1, beträgt.

Zweckmäßig wird das Reaktionsgemisch durch die alkalisch reagierende wäßrige Lösung auf einen pH-Bereich von 7,0 bis 11,5, vorzugsweise 8 bis 11, eingestellt.

Nach einer weiteren Ausführungsform wird das Reaktionsgemisch auf Raumtemperatur abgekühlt und die Hauptmenge des ausgefällten Salzes abgetrennt, vorzugsweise abfiltriert.

Das Molverhältnis von Glycerin zu Epichlorhydrin beträgt 10 : 1 bis 1 : 1, vorzugsweise 6 : 1 bis 1,4 : 1. Zweckmäßig beträgt das Molverhältnis von Glycerin zu Epichlorhydrin nach einer Ausführungsform zur Erhöhung des Diglycerinanteiles 3 : 1 bis 1 : 1, vorzugsweise 2,0 : 1 bis 1,5 : 1.

Nach einer anderen Ausführungsform wird die alkalisch reagierende Alkalicarbonatlösung dem nicht aufgetrennten Reaktionsgemisch aus der Umsetzung von Epichlorhydrin mit Glycerin in geringem Überschuß zugegeben und nach beendeter Reaktion dieser Überschuß neutralisiert.

Zur Neutralisation wird bevorzugt Salzsäure eingesetzt, jedoch können auch andere Mineralsäuren oder saure Kationenaustauscher verwendet werden.

Erfindungsgemäß wurde weiterhin festgestellt, daß mit der Änderung der Molverhältnisse eine Änderung der Reaktionszeit einhergeht. Es ergab sich, daß hinsichtlich der Reaktionszeit, des hohen Anteils an Diglycerin und des gewünschten minimalen Gehalts der Roh-Polyglycerinmischung an cyclischen Komponenten ein Molverhältnis von Glycerin zu Epichlorhydrin von 2,0 bis 1,5 : 1 sich als besonders günstig erwies.

Die Regenerierung der Kationenaustauschermasse in den Kationenaustauschern erfolgt bevorzugt mittels einer Gleichstrom- oder Verbund-Gleichstrom-Regenerierung.

Nach einer bevorzugten Ausführungsform werden nach der Regenerierung die Salze ausgewaschen. Nach Beeindigung des Auswaschvorganges wird die polyglycerinhaltige, vorzugsweise diglycerinhaltige Lösung durch die Ionenaustauscher durchgeleitet und die den Anionenaustauscher verlassende polyglycerinhaltige, vorzugsweise diglycerinhaltige Lösung bis zur Erreichung eines Polyglyceringehaltes, vorzugsweise Diglyceringehaltes, von 20 Gew.-%, vorzugsweise bis zur Erreichung eines Polyglyceringehaltes, vorzugsweise Diglyceringehaltes, von 15 Gew.-%, zurückgeleitet und zur Herstellung der 70 - 40 gew.-%igen, vorzugsweise 60 - 50 gew.-%igen, polyglycerinhaltigen, vorzugsweise diglycerinhaltigen Ausgangslösung mitverwendet.

Die Durchleitung der polyglycerinhaltigen, vorzugsweise diglycerinhaltigen Lösung erfolgt durch die Ionenaustauscher bevorzugt unter einem Überdruck.

Die polyglycerinhaltige, vorzugsweise diglycerinhaltige Lösung wird dabei unter einem Druck von 1,1 - 8 bar, vorzugsweise 1,5 - 5 bar, durch die Ionenaustauscher, d. h. durch einen oder mehreren Kationenaustauscher und mindestens einen Anionenaustauscher, geleitet. Zur Steuerung und Aufrechterhaltung des Druckes sind an einer oder mehreren Stellen in der Leitung oder an den Ionenaustauschern Ventile angebracht.

Dabei wird zweckmäßig die polyglycerinhaltige, vorzugsweise diglycerinhaltige Lösung mit einer Strömungsgeschwindigkeit von 0,5 m/h bis 15 m/h, vorzugsweise 1m/h bis 5m/h, durch die Ionenaustauscher geleitet.

Als Kationenaustauschermassen und Anionenaustauschermassen werden bevorzugt solche verwendet, die temperaturbeständig bis über 80 °C, vorzugsweise bis über 100 °C, sind.

Die Ionenaustauschermasse des Kationenaustauschers und/oder Anionenaustauschers ist zweckmäßig von einer Siebplatte, Lochplatte oder einer in der Höhenrichtung des Ionenaustauschers verschiebbar angeordneten, die Austauschermasse abdeckenden und einen gleichmäßigen Flüssigkeitsdurchtritt ermöglichenden Vorrichtung und/oder einer inerten Preßmasse und/oder elastischen Kunststoffmasse bedeckt.

Die stark saure Kationenaustauschermasse und die schwach basische Anionenaustauschermasse besitzen bevorzugt eine innere Oberfläche (gemessen nach Methode BET) von mehr als 25 $m^2/g$, vorzugsweise 50 bis 100 $m^2/g$.

Das erfindungsgemäße Verfahren kann kontinuierlich oder diskontinuierlich durchgeführt werden.

Ausführungsbeispiele:

Beispiel 1

Herstellung von Polyglycerin mit einem höheren Anteil an Diglycerin:

0,952 kg (10 mol) Glycerin und 3,5 ml konzentrierte Schwefelsäure werden in einem 2-1-Doppelwandreaktor (Thermoflüssigkeit: Öl) vorgelegt. Unter Rühren werden 0,639 kg (6,9 mol) Epichlorhydrin so schnell zugetropft, daß die Reaktionstemperatur unter 90 °C bleibt (gegebenenfalls wird das Thermoöl über einen Wärmeaustauscher abgekühlt). Nach ca. 2-stündiger Zugabe wird der Ansatz zur vollständigen Abreaktion noch weitere 15 min gerührt.

Rohauswaage: 1,5 kg

1,898 l einer 2-molaren Sodalösung (entsprechend dem Gehalt an organisch gebundenem Chlor aus vorgenannter Umsetzung plus 10 % Überschuß) werden auf ca. 90 °C erhitzt. Unter Rühren wird die nicht weiter aufgearbeitete Chlorhydrinethermischung innerhalb von 2 h zu dieser alkalischen Lösung zugetropft. Nach weiteren 30 min bei dieser Temperatur wird die Heizung abgestellt und der Reaktionsansatz durch Zugabe 1/2 konzentrierten Salzsäure neutralisiert. Die neutrale Reaktionslösung wird im Vakuum eingeengt, die ausgefallenen Salze abfiltriert und das Filtrat nach Verdünnung mit Wasser über eine Kombination von Kationen- und Anionenaustauschern entsalzt. Zur Entwässerung wird diese Roh-Polyglycerinlösung im Vakuum eingedampft.

Das Produktgemisch hatte folgende Zusammensetzung (in Gew.-%): Glycerin 36,3 Gew.-%, cycl. Diglycerin 0,9 Gew.-%, Diglycerin 42,0 Gew.-% cycl. Triglycerin 0,4 Gew.-%, Triglycerin 15,2 Gew.-%, cycl. Tetraglycerin 0,3 Gew.-%, Tetraglycerin 4,5 Gew.-%, Pentaglycerin 0,4 Gew.-%.

Beispiel 2

Herstellung von Polyglycerin mit einem höheren Anteil an Diglycerin entsprechend Ausführungsbeispiel 1, jedoch in einem Molverhältnis von Glycerin zu Epichlorhydrin von 5 : 1. Das erhaltene Produktgemisch hatte folgende Zusammensetzung (in Gew.-%): Glycerin 60 Gew.-%, cycl. Diglycerin 1,4 Gew.-%, Diglycerin 29,7 Gew.-%, cycl. Triglycerin 0,6, Tetraglycerin 0,8 Gew.-%.

Beispiel 3

Ausführungsbeispiel 3 wie Beispiel 1, jedoch in einem Molverhältnis von Glycerin zu Epichlorhydrin von 2 : 1. Das erhaltene Produktgemisch enthielt als Hauptbestandteil Glycerin 37,5 Gew.-%, Diglycerin 40,8 Gew.-%, cycl. Diglycerin 1,0 Gew.-% und anderen Nebenbestandteilen.

## Patentansprüche

1. Verfahren zur Herstellung von Polyglycerinen (mit mehr als 50 Gew.-% Diglycerin), die arm an cyclischen Komponenten sind, durch Umsetzung von Glycerin mit Chlorhydrinen, dadurch gekennzeichnet, daß Glycerin mit Epichlorhydrin (anstelle von Chlorhydrin) bei Temperaturen von 20 bis 140°C in Gegenwart eines sauren Katalysators und in einem Molverhältnis von Glycerin zu Epichlorhydrin von 10 : 1 bis 1 : 1 umgesetzt und das erhaltene, nicht aufgetrennte Reaktionsgemisch und/oder das weitgehend von überschüssigem Glycerin befreite Reaktionsgemisch bei einer Temperatur von 50 °C bis 120°C, entsprechend dem Gehalt des Reaktionsgemisches an organisch gebundenem Chlor mit einem alkalisch reagierenden Medium umgesetzt wird, das Reaktionsgemisch nach Wasserzugabe über einen oder mehrere Kationenaustauscher und nachfolgend unter Verwendung von Anionenaustauschern entsalzt, durch Destillation entwässert und das Glycerin-Polyglyceringemisch durch fraktionierte Destillation in Glycerin, Diglycerin und höhere Polyglycerine aufgetrennt wird.

2. Verfahren nach Anspruch 1, dadurch gekennzeichnet, daß bei der Umsetzung von Glycerin als saurer Katalysator eine Säure, vorzugsweise Schwefelsäure, Perchlorsäure, Phosphorsäure und/oder phosphorige Säure und/oder Lewis-Säure, verwendet wird.

3. Verfahren nach Ansprüchen 1 und 2, dadurch gekennzeichnet, daß die Umsetzung von Glycerin mit Epichlorhydrin bei Temperaturen von 60 °C bis 100 °C in einem Molverhältnis von Glycerin zu Epichlorhydrin von 6 : 1 bis 1,4 : 1 erfolgt und das Reaktionsgemisch bei einer Temperatur von 60 bis 95 °C mit einer alkalisch reagierenden wäßrigen Lösung umgesetzt wird.

4. Verfahren nach einem oder mehreren der Ansprüche 1 bis 3, dadurch gekennzeichnet, daß die als saurer Katalysator verwendete Säure in einer Konzentration von 0,1 bis 2,0 Gew.-%, vorzugsweise von 0,5 bis 1,2 Gew.-%, bezogen auf das eingesetzte Epichlorhydrin zugesetzt wird.

5. Verfahren nach einem oder mehreren der Ansprüche 1 bis 4, dadurch gekennzeichnet, daß das Reaktionsgemisch durch Wasserzugabe auf eine 70 - 40gew.-%ige Lösung, vorzugsweise 60 - 50 gew.%ige Lösung, verdünnt und bei Temperaturen von 30 °C bis 90 °C, vorzugsweise von 40 °C bis 70°C, über eine Kombination von stark saurem Kationen- und nachfolgenden schwach basischen Anionenaustauscher entsalzt wird.

6. Verfahren nach einem oder mehreren der Ansprüche 1 bis 5, dadurch gekennzeichnet, daß das nicht aufgetrennte Reaktionsgemisch aus der Umsetzung von Epichlorhydrin mit Glycerin zu einer alkalisch reagierenden Alkalicarbonatlösung, vorzugsweise einer konzentrierten Sodalösung, gegeben wird.

7. Verfahren nach einem oder mehreren der Ansprüche 1 bis 6, dadurch gekennzeichnet, daß das nicht aufgetrennte Reaktionsgemisch aus der Umsetzung von Epichlorhydrin mit Glycerin zu einer alkalisch reagierenden Alkalicarbonatlösung gegeben wird, wobei das äquivalente Verhältnis von Alkalicarbonat zu dem Gehalt an organisch gebundenem Chlor
   1 : 1 bis 1,2 : 1, vorzugsweise
   1,05 : 1 bis 1,1 : 1,
beträgt.

8. Verfahren nach einem oder mehreren der Ansprüche 1 bis 7, dadurch gekennzeichnet, daß die alkalisch reagierende Alkalicarbonatlösung dem nicht aufgetrennten Reaktionsgemisch aus der Umsetzung von Epichlorhydrin mit Glycerin in geringem Überschuß zugegeben und nach beendeter Reaktion dieser Überschuß neutralisiert wird.

9. Verfahren nach einem oder mehreren der Ansprüche 1 bis 8, dadurch gekennzeichnet, daß das Reaktionsgemisch durch die alkalisch reagierende wäßrige Lösung auf einen pH-Bereich von
   7,0 bis 11,5, vorzugsweise
   8 bis 11 ,
   eingestellt wird.

10. Verfahren nach einem oder mehreren der Ansprüche 1 bis 9, dadurch gekennzeichnet, daß das Reaktionsgemisch auf Raumtemperatur abgekühlt und die Hauptmenge des ausgefällten Salzes abgetrennt, vorzugsweise abfiltriert wird.

11. Verfahren nach einem oder mehreren der Ansprüche 1 bis 10, dadurch gekennzeichnet, daß das Molverhältnis von Glycerin zu Epichlorhydrin zur Erhöhung des Diglycerinanteiles
   10 : 1 bis 1 : 1, vorzugsweise
   6,0 : 1 bis 1,4 : 1,
beträgt.

12. Verfahren nach einem oder mehreren der Ansprüche 1 bis 11, dadurch gekennzeichnet, daß die Regenerierung der Kationenaustauschermasse in den Kationenaustauschern mittels einer Gleichstrom- oder Verbund-Gleichstrom-Regenerierung erfolgt.

13. Verfahren nach einem oder mehreren der Ansprüche 1 bis 12, dadurch gekennzeichnet, daß nach der Regenerierung die Salze ausgewaschen, nach Beendigung des Auswaschvorganges die polyglycerinhaltige, vorzugsweise diglycerinhaltige Lösung durch die Ionenaustauscher durchgeleitet wird und die den Anionenaustauscher verlassende polyglycerinhaltige, vorzugsweise diglycerinhaltige Lösung bis zur Erreichung eines Polyglyceringehaltes, vorzugsweise Diglyceringehaltes, von 20 Gew.-%, vorzugweise bis zur Erreichung eines Polyglyceringehaltes, vorzugsweise Diglyceringehaltes, von 15 Gew.-%, zurückgeleitet und zur Herstellung der
    70 - 40 gew.-%igen, vorzugsweise
    60 - 50 gew.-%igen,
polyglycerinhaltigen, vorzugsweise diglycerinhaltigen Ausgangslösung mitverwendet wird.

14. Verfahren nach einem oder mehreren der Ansprüche 1 bis 13, dadurch gekennzeichnet, daß die Durchleitung der polyglycerinhaltigen, vorzugsweise diglycerinhaltigen Lösung durch die Ionenaustauscher unter Überdruck erfolgt.

15. Verfahren nach einem oder mehreren der Ansprüche 1 bis 14, dadurch gekennzeichnet, daß die polyglycerinhaltige, vorzugsweise diglycerinhaltige Lösung unter einem Druck von
    1,1 - 8 bar, vorzugsweise
    1,5 - 5 bar,
durch einen oder mehrere Kationenaustauscher und mindestens einen Anionenaustauscher geleitet wird.

16. Verfahren nach einem oder mehreren der Ansprüche 1 bis 15, dadurch gekennzeichnet, daß die Ionenaustauschermasse des Kationenaustauschers und/oder Anionenaustauschers von einer Siebplatte, Lochplatte oder einer in der Höhenrichtung des Ionenaustau schers verschiebbar angeordneten, die Austauschermasse abdeckenden und einen gleichmäßigen Flüssigkeitsdurchtritt ermöglichenden Vorrichtung und-/oder einer inerten Preßmasse und/oder elastischen Kunststoffmasse bedeckt ist.

17. Verfahren nach einem oder mehreren der Ansprüche 1 bis 16, dadurch gekennzeichnet, daß die eingesetzten Kationenaustauschermassen und Anionenaustauschermassen temperaturbeständig
    bis über 80 °C, vorzugsweise
    bis über 100 °C,
sind.

18. Verfahren nach einem oder mehreren der Ansprüche 1 bis 17, dadurch gekennzeichnet, daß die stark saure Kationenaustauschermasse und die schwach basische Anionenaustauschermasse eine innere Oberfläche (gemessen nach Methode BET) von
    mehr als 25 m²/, vorzugsweise
    50 bis 100 m²/g,
aufweisen.

19. Verfahren nach einem oder mehreren der Ansprüche 1 bis 18, dadurch gekennzeichnet, daß die polyglycerinhaltige, vorzugsweise diglycerinhaltige Lösung mit einer Strömungsgeschwindigkeit von
    0,5 m/h bis 15 m/h, vorzugsweise
    1 m/h bis 5 m/h,
durch die Ionenaustauscher geleitet wird.

## Claims

1. A method for preparing polyglycerols (containing more than 50% by weight diglycerol) which are low in cyclic constituents, by reacting glycerol with chlorohydrins, characterised in that glycerol is reacted with epichlorohydrin (instead of chlorohydrin) at temperatures of 20 to 140°C in the presence of an acidic catalyst and in a molar ratio of glycerol to epichlorohydrin of 10 : 1 to 1 : 1, and the resulting, non-separated reaction mixture and/or the reaction mixture which is largely freed of excess glycerol is reacted with an alkaline-reacting medium at a temperature of 50°C to 120°C, according to the content of organically bound chlorine of the reaction mixture, the reaction mixture is desalted after the addition of water via one or more cation exchangers and then using anion exchangers, is dehydrated by distillation and the glycerol-polyglycerol mixture is separated into glycerol, diglycerol and higher polyglycerols by fractional distillation.

2. A method according to Claim 1, characterised in that an acid, preferably sulphuric acid, perchloric acid, phosphoric acid and/or phosphorous acid and/or Lewis acid is used as an acidic catalyst during the reaction of glycerol.

3. A method according to Claims 1 and 2, characterised in that the reaction of glycerol with epichlorohydrin takes place at temperatures of 60°C to 100°C in a molar ratio of glycerol to epichlorohydrin of 6 : 1 to 1.4 : 1 and the reaction mixture is reacted with an alkaline-reacting aqueous solution at a temperature of 60 to 95°C.

4. A method according to one or more of Claims 1 to 3, characterised in that the acid used as an acidic catalyst is added in a concentration of 0.1 to 2.0% by weight, preferably of 0.5 to 1.2% by weight, relative to the epichlorohydrin used.

5. A method according to one or more of Claims 1 to 4, characterised in that the reaction mixture is diluted by the addition of water to form a 70 - 40% by weight solution, preferably 60 - 50% by weight solution, and is desalted at temperatures of 30°C to 90°C, preferably of 40°C to 70°C, via a combination of strongly acidic cation exchanger and subsequent weakly basic anion exchanger.

6. A method according to one or more of Claims 1 to 5, characterised in that the non-separated reaction mixture from the reaction of epichlorohydrin with glycerol is added to an alkaline-reacting alkali carbonate solution, preferably a concentrated soda solution.

7. A method according to one or more of Claims 1 to 6, characterised in that the non-separated reaction mixture from the reaction of epichlorohydrin with glycerol is added to an alkaline-reacting alkali carbonate solution, the equivalent ratio of alkali carbonate to the content of organically bound chlorine being
1 : 1 to 1.2 : 1, preferably
1.05 : 1 to 1.1 : 1.

8. A method according to one or more of Claims 1 to 7, characterised in that the alkaline-reacting alkali carbonate solution is added to the non-separated reaction mixture from the reaction of epichlorohydrin with glycerol in a slight excess and this excess is neutralised once the reaction has ended.

9. A method according to one or more of Claims 1 to 8, characterised in that the reaction mixture is set by the alkaline-reacting aqueous solution to a pH range of
7.0 to 11.5, preferably
8 to 11.

10. A method according to one or more of Claims 1 to 9, characterised in that the reaction mixture is cooled to room temperature and the greater part of the precipitated salt is separated off, preferably filtered off.

11. A method according to one or more of Claims 1 to 10, characterised in that the molar ratio of glycerol to epichlorohydrin is
10 : 1 to 1 : 1, preferably
6.0 : 1 to 1.4 : 1,
in order to increase the diglycerol content.

12. A method according to one or more of Claims 1 to 11, characterised in that the regeneration of the cation exchanger substance in the cation exchangers takes place by means of direct current or composite-direct current regeneration.

13. A method according to one or more of Claims 1 to 12, characterised in that after the regeneration the salts are washed out, after the washing-out operation has finished the polyglycerol-containing, preferably diglycerol-containing, solution is passed through the ion exchanger and the polyglycerol-containing, preferably diglycerol-containing, solution leaving the anion exchanger is fed back until a polyglycerol content, preferably diglycerol content, of 20% by weight is achieved, preferably until a polyglycerol content, preferably diglycerol content, of 15% by weight is achieved, and is jointly used to produce the
70 - 40% by weight, preferably
60 - 50% by weight,
polyglycerol-containing, preferably diglycerol-containing, starting solution.

14. A method according to one or more of Claims 1 to 13, characterised in that the polyglycerol-containing, preferably diglycerol-containing, solution is passed through the ion exchangers under excess pressure.

15. A method according to one or more of Claims 1 to 14, characterised in that the polyglycerol-containing, preferably diglycerol-containing, solution is passed through one or more cation exchangers and at least one anion exchanger at a pressure of
1.1 - 8 bar, preferably
1.5 - 5 bar.

16. A method according to one or more of Claims 1 to 15, characterised in that the ion exchanger substance of the cation exchanger and/or anion exchanger is covered by a sieve plate, perforated plate or a device which is arranged to be displaceable in the vertical direction of the ion exchanger, covers the exchanger substance and permits even penetration by liquid, and/or an inert moulding compound and/or elastic plastic substance.

17. A method according to one or more of Claims 1 to 16, characterised in that the cation exchanger substances and anion exchanger substance used are heat-resistant to
above 80°C, preferably
above 100°C.

18. A method according to one or more of Claims 1 to 17, characterised in that the strongly acidic cation exchanger substance and the weakly basic anion exchanger substance have an internal surface area (measured according to the BET method) of
more than 25 m²/g, preferably
50 to 100 m²/g.

19. A method according to one or more of Claims 1 to 18, characterised in that the polyglycerol-containing, preferably diglycerol-containing, solution is passed through the ion exchanger at a flow rate of
0.5 m/h to 15 m/h, preferably
1 m/h to 5 m/h.

## Revendications

1. Procédé de préparation de polyglycérines (avec plus de 50 % en poids de diglycérine), qui sont pauvres en composants cycliques, par réaction de la glycérine avec des chlorhydrines, caractérisé en ce qu'on fait réagir de la glycérine avec de l'épichlorydrine (au lieu de chlorhydrine) à des températures de 20 à 140°C, en présence d'un catalyseur acide et dans un rapport molaire de la glycérine à l'épichlorhydrine de 10 : 1 à 1 : 1, et on fait réagir le mélange réactionnel non séparé obtenu et/ou le mélange réactionnel largement débarrassé de la glycérine en excès à une température de 50 à 120°C, selon la teneur du mélange réactionnel en chlore organique lié, avec un milieu alcalin, on déminéralise le mélange réactionnel après addition d'eau via un ou plusieurs échangeurs de cations et, ensuite, par l'emploi d'échangeurs d'anions, on déshydrate par distillation et on sépare par distillation fractionnée le mélange de glycérine et de polyglycérines en glycérine, diglycérine et polyglycérines supérieures.

2. Procédé selon la revendication 1, caractérisé en ce qu'on utilise comme catalyseur acide pour la conversion de la glycérine un acide, de préférence l'acide sulfurique, l'acide perchlorique, l'acide phosphorique et/ou un acide contenant du phosphore et/ou un acide de Lewis.

3. Procédé selon les revendications 1 et 2, caractérisé en ce que la réaction de la glycérine et de l'épichlorhydrine se fait à des températures de 60 à 100°C selon un rapport molaire de la glycérine à l'épichlorhydrine de 6 : 1 à 1,4 : 1 et le mélange réactionnel est mis en réaction avec une solution aqueuse alcaline à une température de 60 à 95°C.

4. Procédé selon une ou plusieurs des revendications 1 à 3, caractérisé en ce que l'acide utilisé à titre de catalyseur acide sera ajouté en concentration de 0,1 à 2,0 % en poids, de préférence de 0,5 à 1,2 % en poids, par rapport à l'épichlorhydrine utilisée.

5. Procédé selon une ou plusieurs des revendications 1 à 4, caractérisé en ce que le mélange réactionnel est dilué par addition d'eau pour obtenir une solution à environ 70-40 % en poids, de préférence 60-50 % en poids et déminéralisé à des températures de 30 à 90°C, de préférence 40 à 70°C, via une combinaison d'échangeurs de cations fortement acides et, ensuite, d'échangeurs d'anions faiblement basiques.

6. Procédé selon une ou plusieurs des revendications 1 à 5, caractérisé en ce que le mélange réactionnel non séparé formé par réaction de l'épichlorhydrine avec la glycérine est ajouté à une solution alcaline de carbonate de métal alcalin, de préférence une solution de soude concentrée.

7. Procédé selon une ou plusieurs des revendications 1 à 6, caractérisé en ce que le mélange réactionnel non séparé formé par réaction de l'épichlorhydrine avec la glycérine est ajouté à une solution alcaline de carbonate de métal alcalin dans laquelle le rapport équivalent du carbonate de métal alcalin à la teneur en chlore organique lié atteint 1 : 1 à 1,2 : 1, de préférence 1,05 : 1 à 1,1 : 1.

8. Procédé selon une ou plusieurs des revendications 1 à 7, caractérisé en ce que la solution alcaline de carbonate de métal alcalin est ajoutée en faible excès au mélange réactionnel non séparé formé par réaction de l'épichlorhydrine avec la glycérine et, une fois la réaction terminée, cet excès est neutralisé.

9. Procédé selon une ou plusieurs des revendications 1 à 8, caractérisé en ce que le mélange réactionnel est ajusté par la solution aqueuse à réaction alcaline dans une plage de pH de 7,0 à 11,5, de préférence 8 à 11.

10. Procédé selon une ou plusieurs des revendications 1 à 9, caractérisé en ce que le mélange réactionnel est refroidi à température ambiante et la quantité principale du sel précipité est séparée, de préférence par filtration.

11. Procédé selon une ou plusieurs des revendications 1 à 10, caractérisé en ce que le rapport molaire de la glycérine à l'épichlorhydrine pour augmenter la fraction de diglycérine est de 10 : 1 à 1 : 1, de préférence 6,0 : 1 à 1,4 : 1.

12. Procédé selon une ou plusieurs des revendications 1 à 11, caractérisé en ce que la régénération de la masse échangeuse de cations dans les échangeurs de cations se fait de préférence par régénération en courant continu ou par régénération en courant continu combiné.

13. Procédé selon une ou plusieurs des revendications 1 à 12, caractérisé en ce que, après régénération, les sels sont éliminés par lavage, à la fin de l'opération de lavage, la solution contenant des polyglycérines, de préférence de la diglycérine, est envoyée à travers l'échangeur d'ions et la solution contenant des polyglycérines, de préférence de la diglycérine quittant l'échangeur d'anions est renvoyée dans le circuit jusqu'à atteindre un teneur en polyglycérines, de préférence une teneur en diglycérine, de 20 % en poids, de préférence jusqu'à atteindre une teneur en polyglycérines, de préférence une teneur en diglycérine, de 15 % en poids et est utilisée conjointement pour la préparation de la solution de départ contenant 70 à 40 % en poids, de préférence 60 à 50 % en poids de polyglycérines, de préférence de diglycérine.

14. Procédé selon une ou plusieurs des revendications 1 à 13, caractérisé en ce que le passage à travers les échangeurs d'ions de la solution contenant des polyglycérines, de préférence de la diglycérine se fait sous pression.

15. Procédé selon une ou plusieurs des revendications 1 à 14, caractérisé en ce que la solution contenant des polyglycérines, de préférence de la diglycérine, est de préférence envoyée sous une pression de 1,1 à 8 bars, de préférence 1,5 à 5 bars à travers un ou plusieurs échangeurs de cations et au moins un échangeur d'anions.

16. Procédé selon une ou plusieurs des revendications 1 à 15, caractérisé en ce que la masse échangeuse d'ions de l'échangeur de cations et/ou de l'échangeur d'anions sera recouverte de manière appropriée par une plaque-tamis, une plaque perforée ou un dispositif et/ou une masse moulée inerte et/ou une masse de matière plastique élastique agencés de manière à pouvoir se déplacer dans le sens de la hauteur de l'échangeur d'ions, recouvrant la masse échangeuse et permettant un passage uniforme du liquide.

17. Procédé selon une ou plusieurs des revendications 1 à 16, caractérisé en ce que les masses échangeuses de cations et d'anions utilisées résistent à la chaleur jusqu'à plus de 80°C, de préférence jusqu'à plus de 100°C.

18. Procédé selon une ou plusieurs des revendications 1 à 17, caractérisé en ce que la masse échangeuse de cations fortement acide et la masse échangeuse d'anions faiblement basique possèdent une surface interne (mesurée par la méthode BET) de plus de 25 $m^2$/g, de préférence de 50 à 100 $m^2$/g.

19. Procédé selon une ou plusieurs des revendications 1 à 18, caractérisé en ce que la solution contenant des polyglycérines, de préférence de la diglycérine est envoyée à travers les échangeurs d'ions avec une vitesse d'écoulement de 0,5 m/h à 15 m/h, de préférence 1 m/h à 5 m/h.